# FASCICULE DE BREVET EUROPEEN

(11) **EP 1 461 319 B1**
(45) Date de publication et mention de la délivrance du brevet: **29.04.2009**
(21) Numéro de dépôt: 02795314.0
(22) Date de dépôt: 14.10.2002
(51) Int. Cl.: C07D 215/48, A61K 31/47, A61P 31/18

(54) **DERIVES DE QUINOLEINE, PROCEDE DE SYNTHESE, ET MEDICAMENTS RENFERMANT CES DERIVES**
CHINOLINDERIVATE, SYNTHESEVERFAHREN UND DIESE DERIVATE ENTHALTENDE MEDIKAMENTE
QUINOLINE DERIVATIVES, SYNTHESIS METHOD, AND MEDICINES CONTAINING SAID DERIVATIVES

(30) Priorité: 12.10.2001 FR 0113209
(43) Date de publication de la demande: 29.09.2004
(73) Titulaire: BioAlliance Pharma, 75015 Paris (FR); UNIVERSITE PARIS-SUD (PARIS XI), 91470 Orsay Cedex (FR); INSTITUT NATIONAL DE LA SANTE ET DE LA RECHERCHE MEDICALE (INSERM), 75654 Paris Cédex 13 (FR); Institut Gustave Roussy, 94805 Villejuif Cédex (FR); CENTRE NATIONAL DE LA RECHERCHE SCIENTIFIQUE (CNRS), 75794 Paris Cedex 16 (FR)
(72) Inventeur: D'ANGELO, Jean, F-91300 MASSY (FR); BAYLE, Marie, F-92260 FONTENAY AUX ROSES (FR); BENARD, Christophe, F-91370 VERRIERE LE BUISSON (FR); DESMA LE, Didier, F-94260 FRESNES (FR); DANET, Michèle, F-91440 BURRES SUR YVETTE (FR); JANSON, Laurence, F-94800 VILLEJUIF (FR); LE BRET, Marc, F-75016 Paris (FR); LEH, Hervé, F-75005 PARIS (FR); MOUSCADET, Jean-François, F-92330 SCEAUX (FR); SUBRA, Frédéric, F-75009 PARIS (FR); ZOUHIRI, Fatima, F-92290 CH TENAY-MALABRY (FR)
(74) Mandataire: Domenego, Bertrand
(86) Numéro de dépôt international: PCT/FR2002/003512
(87) Numéro de publication internationale: WO 2003/031413

(56) Documents cités:
- FR-A- 2 761 687
- ZOUHIRI F ET AL: "STRUCTURE-ACTIVITY RELATIONSHIPS AND BINDING MODE OF STYRYLQUINOLINES AS POTENT INHIBITORS OF HIV-1 INTEGRASE AND REPLICATION OF HIV-1 IN CELL CULTURE" JOURNAL OF MEDICINAL CHEMISTRY, AMERICAN CHEMICAL SOCIETY. WASHINGTON, US, vol. 43, 2000, pages 1533-1540, XP000926747 ISSN: 0022-2623

## Description

L'invention a pour objet des dérivés de quinoléine possédant notamment des propriétés inhibitrices des intégrases de rétrovirus. Elle porte également sur un procédé de synthèse de ces dérivés et sur les médicaments les renfermant dans leur principe actif.

Dans la demande WO 98/45269 au nom du CNRS, on a décrit des dérivés de quinoléine possédant des propriétés inhibitrices de l'intégrase du VIH-1. En poursuivant les travaux sur ce type de composés, il est à présent apparu de manière inattendue qu'en élaborant des dérivés de quinoléine renfermant à la fois un groupe identifié comme étant un pharmacophore, en positions 7 et 8 de la quinoléine, et un bras espaceur amide, ou dérivé d'amide, entre la quinoléine et un groupe de substitution du cycle quinoléine, il était possible de disposer d'une nouvelle famille fortement inhibitrice des intégrases rétrovirales.

L'invention a donc pour but de fournir de nouveaux dérivés de quinoléine dotés notamment de propriétés anti-intégrases de rétrovirus.

Elle vise également un procédé de synthèse de ces dérivés.

Selon encore un autre aspect, l'invention porte également sur des compositions pharmaceutiques renfermant ces dérivés.

Les dérivés de quinoléine selon l'invention sont caractérisés en ce qu'ils répondent à la formule I 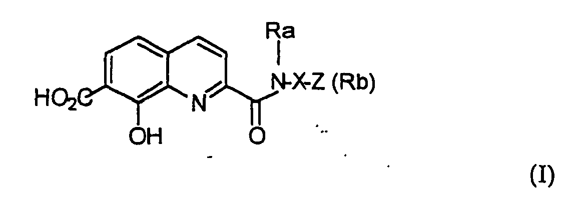 dans laquelle
- X représente une chaîne alkyle-(CH₂)ₙ- dans laquelle n est égal à 0, 1 ou 2, O, ou N,
- Z représente un cycle aromatique pouvant comporter des hétéroatomes choisi parmi O, N ou S, en substitution des atomes de carbone constituant ledit cycle aromatique, ce cycle pouvant ou non être substitué par Rb,
- Rb représente 1 à 3 substituants identiques ou différents, choisis parmi les groupements -OH, -OR, -COOH, -COOR, -COH,
- COR, -NH₂, -NH(R), -NH(R,R'), -SH et -SR et CN,
- Ra représente un atome d'hydrogène ou un groupe -(CH₂)ₙ,-Y, pour lequel n' est égal à 0, 1, 2 ou 3 et Y représente -CH₃,
- COOH, -COOR, -CN, -OH, -OR, SR, ou un groupement aryle éventuellement substitué par Rb,
- R et R', identiques ou différents, représentent une chaîne alkyle linéaire ou ramifiée de 1 à 4 atomes de carbone, et
- leurs sels pharmaceutiquement acceptables.

Des dérivés préférés sont des 7-carboxy-8-hydroxyquinoléines, et leurs sels pharmaceutiquement acceptables, choisis parmi l'acide 2-(3,4-dihydroxy-benzylcarbamoyl)- 8-hydroxyquinoléine-7-carboxylique, l'acide 2-(2,4-dihydroxybenzylcarbamoyl)-8-hydroxy-quinoléine-7-carboxylique, l'acide 2-(2,3,4-trihydroxy-benzylcarbamoyl)-8-hydroxy-quinoléine-7-carboxylique et leurs sels de sodium.

L'invention vise également un procédé de synthèse des dérivés définis ci-dessus.

Ce procédé est caractérisé en ce qu'il comprend la réaction d'une quinoléinecarboxylate de succinimidyle de formule II 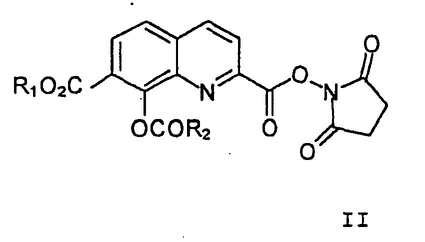 dans laquelle R₁ et R₂, identiques ou différents, représentent des radicaux alkyle de 1 à 4C, avec une amine de formule III 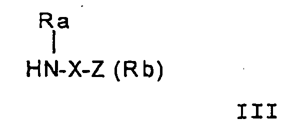 le cas échéant sous forme de sel, suivie d'une saponification et le cas échéant d'une acidification.

Dans un mode de réalisation de l'invention, pour préparer des dérivés de formule IA avec un bras espaceur amide, 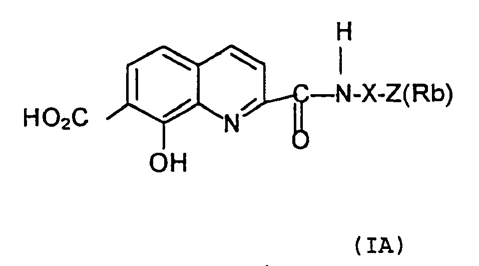 dans laquelle Rb et Z sont tels que définis ci-dessus, et X représente un groupe (CH₂)ₙ- comme défini plus haut, on fait réagir une quinoléinecarboxylate de succinimidyle de formule II avec un sel d'amine de formule IV suivante :

[H₃N-X-Z(Rb)]⁺ A⁻ (IV)

dans laquelle Z et Rb sont tels que définis ci-dessus, X = (CH₂) ₙ comme défini plus haut, et A⁻ est un anion de sel organique, ou un halogénure.

La condensation de la quinoléinecarboxylate de succinimidyle de formule II sur le sel d'amine est effectuée dans la pyridine à température ambiante. En opérant à 20°C environ, le temps de réaction est de l'ordre de 10 à 15 h.

Le sel d'amine est en particulier un para-toluènesulfonate.

L'amide IA est ensuite obtenu par saponification, suivie d'une acidification. La saponification est réalisée par exemple avec une solution de soude 3N. Cette réaction peut être conduite dans le méthanol à température ambiante pendant environ 2 à 4 h, notamment 2 h. Pour l'acidification, on a recours en particulier à de l'acide sulfurique dilué.

Dans un autre mode de réalisation de l'invention, pour préparer des dérivés de formule IB avec un bras espaceur amide substitué, 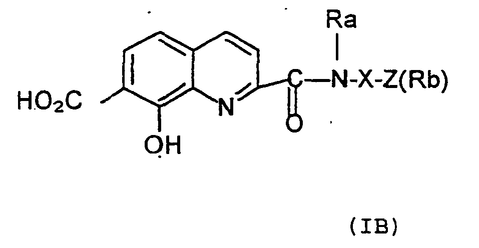 dans laquelle Rb et Z sont tels que définis ci-dessus, et X représente un groupe -(CH₂)ₙ- comme défini plus haut, on fait réagir le dérivé de la quinoléinecarboxylate de succinimidyle de formule II avec une amine de formule V suivante ou son sel organique :

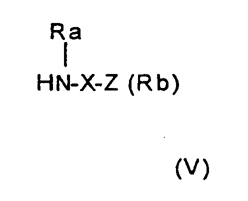

Cette réaction est alors suivie d'une saponification.

Dans un autre mode de réalisation de l'invention, pour préparer des dérivés de formule IC avec un bras espaceur hydrazide, 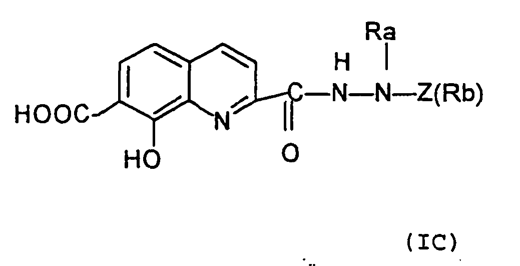 dans laquelle les différents substituants sont tels que définis ci-dessus, on fait réagir la quinoléinecarboxylate de succinimidyle de formule II avec l'hydrazine de formule VI suivante :

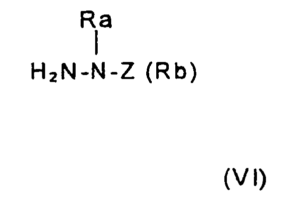

Dans encore un autre mode de réalisation de l'invention, pour préparer des dérivés de formule ID avec un bras espaceur alcoxyamide, 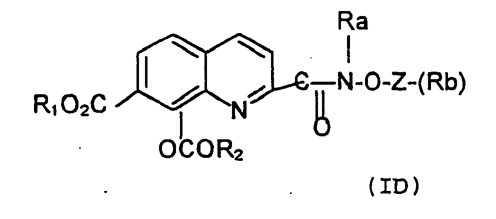 dans laquelle les différents substituants sont tels que définis ci-dessus, on fait réagir la quinoléinecarboxylate de succinimidyle de formule II avec l'hydroxylamine de formule VII suivante :

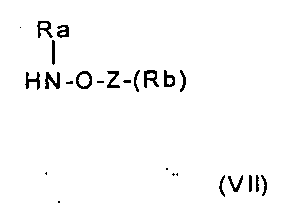

Avantageusement, les dérivés protégés de la quinoléinecarboxylate de succinimidyle de formule II sont obtenus à partir de l'acide 8-hydroxy-7-quinaldine carboxylique de formule VIII :

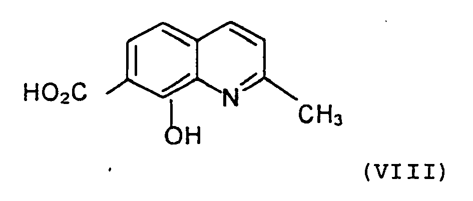

La protection des groupes -COOH et -OH est effectuée à l'aide de dérivés capables d'introduire des groupes protecteurs spécifiques par rapport aux fonctions concernées et pouvant être ensuite coupés sélectivement sans altérer le reste de la molécule.

On procèdera par exemple tout d'abord à l'estérification du groupe -COOH, puis à celle du groupe -OH.

Des résultats satisfaisants ont ainsi été obtenus en soumettant l'hydroxyacide de formule VIII, à l'action d'un alcool aliphatique, comme le méthanol ou le butanol, en présence d'acide polyphosphorique, puis à celle d'un halogénure d'acyle, notamment d'un chlorure d'acyle, comme le chlorure de pivaloyle, en présence de pyridine.

La première réaction d'estérification est conduite à une température de l'ordre de 100°C, pendant environ 50 à 80 h, notamment pendant environ 70 h.

Le traitement de l'ester obtenu avec un halogénure d'acyle est effectué à température ambiante en présence de pyridine, pendant environ 30 min à 2 h, et notamment pendant 1 h.

On procède ensuite à l'oxydation en deux étapes du dérivé diester obtenu. Au cours de la première étape, on oxyde le groupe -CH₃ en C-2 de la quinoléine en groupe -COH, par traitement avec un agent d'oxydation de groupe -CH₃ tel que l'oxyde de sélénium. On opère avantageusement au reflux d'un solvant tel que le dioxane pendant environ 10 à 15 h, notamment 12 h, afin d'obtenir l'aldéhyde correspondant. Au cours d'une deuxième étape, cet aldéhyde est alors oxydé en acide. Des agents d'oxydation appropriés comprennent le chlorite de sodium. En opérant dans un mélange eau/ solvant organique comme le t-butanol, en présence de dihydrogénophosphate de sodium et de 2-méthyl-2-butène, on obtient l'acide correspondant avec un rendement satisfaisant.

Pour préparer la quinoléinecarboxylate de succinimidyle de formule II, on procède à l'activation du groupe -COOH à l'aide, de N-hydroxy-succinimide. La condensation de l'acide avec le N-hydroxy-succinimide est avantageusement réalisée en présence de dicyclohexylcarbodiimide. En opérant à température ambiante, de l'ordre de 20°C, la réaction dure environ 10 à 15 h, notamment 12 h.

L'étude des propriétés des dérivés de l'invention a permis de mettre en évidence leur activité antivirale dirigée contre les intégrases rétrovirales, en général, animales et humaines, et notamment de VIH-1, VIH-2, VIS. VSR. L'efficacité de ces dérivés est observée à des concentrations submicromolaires. Des IC 50 n'excédant pas 1 µM, avantageusement: 0,5µM et même 0.1µM sont ainsi obtenues.

Ces dérivés présentent de plus l'avantage d'un grande innocuité et d'une biodisponibilité satisfaisante de la drogue active.

Ces propriétés sont donc mises à profit conformément à l'invention en utilisant ces dérivés comme principes actifs de médicaments.

L'invention vise donc des compositions pharmaceutiques caractérisées en ce qu'elles comprennent une quantité efficace d'au moins un dérivé tel que défini ci-dessus, en association avec des véhicules pharmaceutiquement acceptables.

Ces compositions sont avantageusement utilisées en combinaison avec d'autres médicaments anti-VIH, en particulier des médicaments dotés d'un effet inhibiteur vis-à-vis de la transcriptase inverse et/ou de la protéase.

Les posologies et les modes d'administration seront adaptés en fonction du traitement par mono ou polythérapie utilisé.

L'invention vise également l'utilisation des dérivés définis ci-dessus en tant que réactifs biologiques utilisables en particulier pour des études de mécanismes concernant l'infection virale.

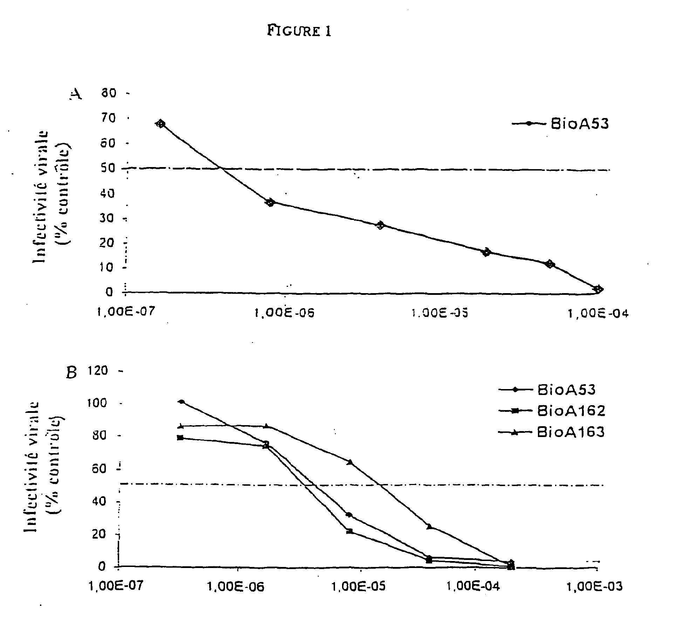

D'autres caractéristiques et avantages de l'invention sont décrits dans les exemples qui suivent, relatifs à la synthèse de dérivés conformes à l'invention et à l'étude de leurs propriétés antivirales dirigées contre les intégrases rétrovirales. Dans ces exemples, il est fait référence aux figures 1 à 3, qui montrent, respectivement :
- les figures 1A et 1B, l'efficacité anti rétrovirale des composés de l'invention,
- les figures 2A et 2B leur absence de toxicité sur des cellules adhérentes et en suspension, et
- les figures 3A et 3B, l'inhibition de la totalité de l'ADN viral intégré (figure 3A) et la détection de l'ADN viral total (figure 3B) .

### I) Synthèse général des amides 10, des hydrazides 12 et des alcoxyamides 14 :

L'hydroxy-acide 1 précédemment décrit dans la littérature¹ est traité à 100 °C pendant 72 heures par le n-butanol en présence d'acide polyphosphorique² pour fournir l'ester butylique 2 . Cet ester traité par le chlorure de pivaloyle dans la pyridine pendant 1 heure à 20 °C donne le diester 3. Celui-ci est oxydé au moyen de dioxyde de sélénium au reflux du dioxanne pendant 12 heures pour donner l'aldéhyde 4, qui est oxydé en acide 5 au moyen de chlorite de sodium dans un mélange eau tert-butanol pendant 12 heures, en présence de dihydrogénophosphate de sodium et de 2-méthyl-2-butène. La quinoléinecarboxylate de succinimidyle 6 est préparée en condensant l'acide 5 sur le N-hydroxy-succinimide en présence de dicyclohexylcarbodiimide pendant 12 heures à 20 °C.

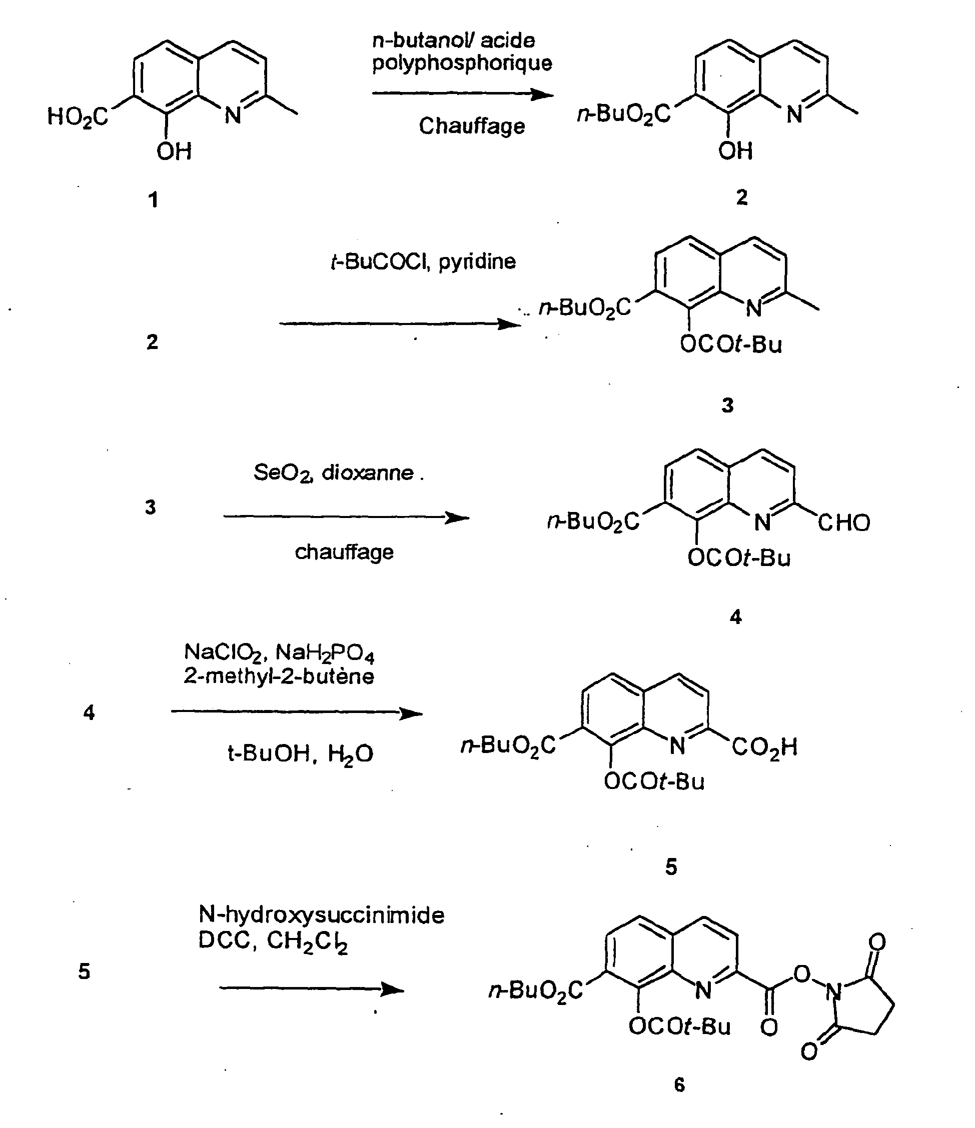

La condensation de la quinoléinecarboxylate de succinimidyle 6 sur le p-toluènesulfonate de 3,4-dihydroxybenzylamine (préparé par hydrogénation catalytique de l'oxime correspondante au moyen du palladium sur charbon, dans l'éthanol en présence d'acide p-toluènesulfonique) dans la pyridine pendant 12 heures à 20° C, fournit l'amide 7, qui par saponification par une solution de soude 3 N, dans le méthanol pendant 3 heures à 20° C, puis acidification à l'acide sulfurique dilué donne l'amide 8.

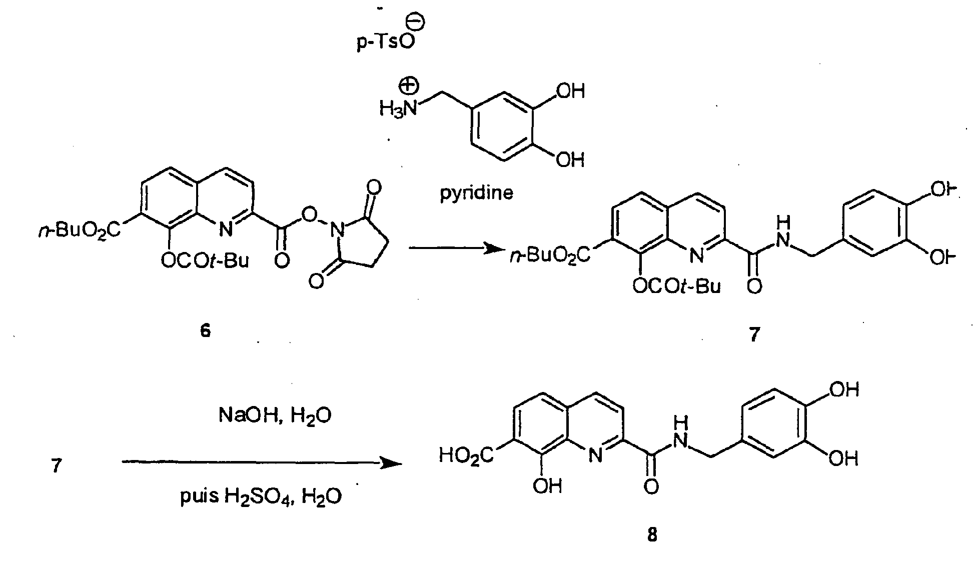

De manière similaire la condensation de 6 avec une amine de formule générale 9 fournit les amides 10. La condensation de 6 avec une hydrazine 11 ou une hydroxylamine 13, suivie de saponification, conduit respectivement aux hydrazides 12 et aux alcoxyamides 14 .

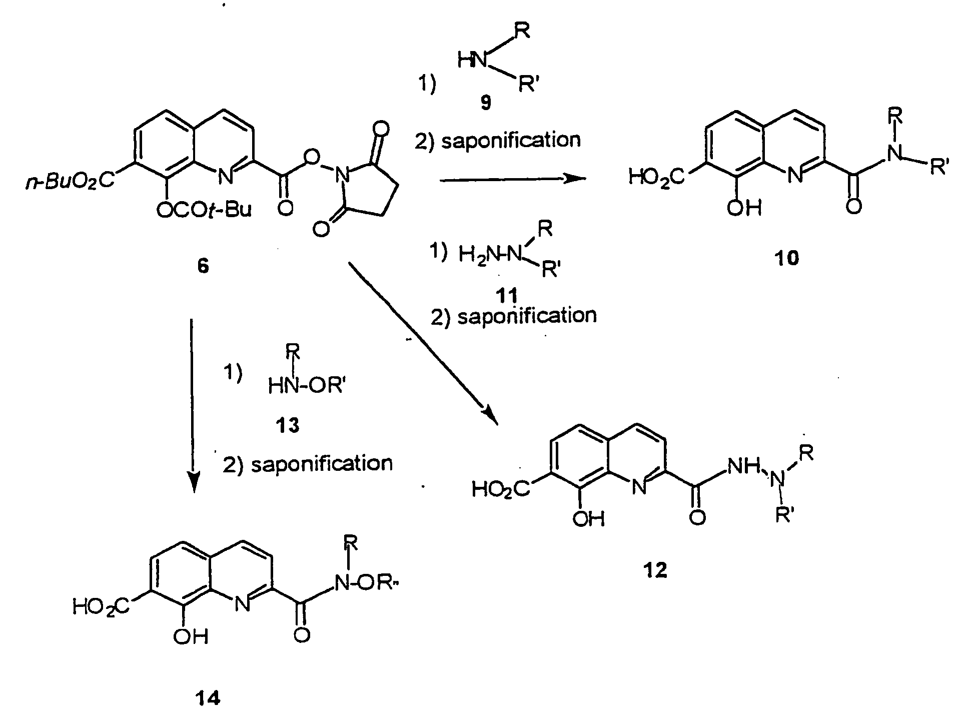

### II. DESCRIPTION DES MODES OPERATOIRES ET DES PRODUITS :

### Synthèse du 2-(3,4-dihydroxy-benzylcarbamoyl)-8-hydroxyquinoléine-7- carboxylate de sodium

### 1^{ère} étape: préparation du 8-hydroxy-2-méthyl-quinoléine-7-carboxylate de n-butyle (2)

Un mélange d'acide 8-hydroxy-7-quinaldine carboxylique 1 (12 g, 59 mmol) et d'acide polyphosphorique (30 g) dans 60 ml de n-butanol est chauffé à 100 °C pendant 72 heures durant lesquelles des portions supplémentaires de 30 g d'acide polyphosphorique et de 30 ml de n-butanol sont ajoutées régulièrement toutes les 12 heures. Après refroidissement à 20 °C, le mélange réactionnel est amené à pH 4 avec une solution d'hydroxyde de potassium 6 N, puis 200 ml de dichlorométhane sont ajoutés. Les phases sont séparées, et la phase aqueuse extraite au dichlorométhane. Les phases organiques rassemblées sont séchées sur sulfate de magnésium et concentrées sous pression réduite. Le résidu est chromatographié sur colonne de silice en éluant par un mélange: cyclohexane / acétate d'éthyle 1 / 1 pour donner 11 g (72%) de 8-hydroxy-2-méthyl-quinoléine-7-carboxylate de n-butyle 2 sous la forme d'un solide blanc.
F = 50 °C
Rf = 0,63 (acétate d'éthyle)
IR (pur, cm⁻¹) ν : 1650 ; 2959
RMN¹H (δ en ppm, CDCl₃, 200 MHz) 0,90 (t, *J*=7,4 Hz, 3H); 1,41 (hex, *J*=7,4 Hz, 2H); 1,70 (quint, *J*=6,7 Hz, 2H); 2,68 (s, 3H); 4, 32 (t, *J*=6, 7 Hz, 2H); 7,09 (d, *J*=8,7 Hz, 1H) ; 7,26 (d, *J*=8, 5 Hz, 1H) ; 7,70 (d, *J*=8,7 Hz, 1H) ; 7,86 (d, *J*=8,5 Hz, 1H) RMN¹³C (δ en ppm, CDCl₃, 50 MHz) : 13,6; 19,1; 25,3; 30,5 ; 65,3; 109,3; 117,3; 124,3; 124,6; 130,3; 135,6; 139,0; 158,4; 159,3; 170,4.
Microanalyse: (C₁₅H₁₇NO₃)
Théorie: C 69,48%; H 6,61%; N 5,40%
Exp. : C 69,33%; H 6,77%; N 5,37%

### 2^{ème} étape : préparation du 8- (2, 2-diméthylpropionyloxy) -2-méthyl-quinoléine-7-carboxylate de n-butyle (3).

A une solution de l'ester précédent 2 (étape 1) (11,0 g, 42 mmol) dans la pyridine (45 ml), on additionne, à 0 °C, 10,4 ml (84 mmol) de chlorure de pivaloyle. Après 1 heure à 20 °C, la pyridine est distillée sous pression réduite, puis 20 ml d'eau et 50 ml de dichlorométhane sont ajoutés. Les phases sont séparées et la phase aqueuse extraite au dichlorométhane. Les phases organiques rassemblées sont séchées sur sulfate de magnésium et concentrées sous pression réduite. Le résidu est chromatographié sur colonne de silice en éluant par un mélange: cyclohexane / acétate d'éthyle 2/1 pour donner 11,7 g (82%) de 8-(2,2-diméthylpropionyloxy)-2-méthyl-quinoléine-7-carboxylate de n-butyle 3 sous forme d'un solide jaune .
F = 52 °C
Rf = 0,88 (acétate d'éthyle)
IR (pur, cm⁻¹) v: 1725; 1753;
RMN¹H (δ en ppm, CDCl₃) , 200MHz) 0,86 (t, *J*=7,5 Hz, 3H) ; 1,36(hex, *J*=7,5 Hz, 2H) ; 1,46(s, 9H) ; 1,65 (quint, *J*=6,8 Hz, 2H) ; 2,52 (s, 3H) ; 4,24 (t, *J*=6,7 Hz, 2H) ; 7,17 (d, *J*=8,4 Hz, 1H) ; 7,48 (d, *J*=8,4 Hz, 1H); 7,84 (d, *J*=8,6 Hz, 2H)
Microanalyse : (C₂₀H₂₅NO₄)
Théorie : C 69,95%; H 7,34%; N 4,08%
Exp. : C 70,04%; H 7,40%; N 3,99%

### 3^{ème} étape : préparation du 8-(2,2-diméthyl-propionyloxy)-2-formyl-quinoléine-7-carboxylate de n-butyle (4)

Un mélange du diester précédent 3 (étape 2) (11,5 g, 34 mmol) et de dioxyde de sélénium (6,7 g, 60 mmol) dans 50 ml de dioxane-1,4 est porté à reflux pendant 12 heures. Après refroidissement à 20 °C, le mélange réactionnel est filtré sur Célite® et concentré sous pression réduite. Le résidu est chromatographié sur colonne de silice en éluant par un mélange: cyclohexane / acétate d'éthyle 4 / 1 pour donner 8,6 g (71%) de 8-(2,2-diméthyl-propionyloxy)-2-formylquinoléine-7-carboxylate de n-butyle 4. Solide blanc.
F = 108 °C
Rf = 0,88 (acétate d'éthyle)
IR (pur, cm⁻¹) v: 1712; 1729; 1752.
RMN¹H (δ en ppm, CDCl₃, 200mhz) 0,90 (t, *J*=7,3 Hz, 3H) ; 1,41 (hex, *J*=7,3 Hz, 2H) ; 1,50(s, 9H) ; 1,72(quint, *J*=6,7 Hz, 2H) ; 4,31 (t, *J*=6,7 Hz, 2H) ; 7,71 (d, *J*=8,7 Hz, 1H) ; 8,00 (d, *J*=8,4 Hz, 1H) ; 8,09 (d, *J*=8,7 Hz, 1H) ; 8,24 (d, *J*=8,4 Hz, 1H) ; 10,05 (s, 1H)
RMN¹³C(δ en ppm, CDCl₃, 50mhz) 13,7 ; 19,2 ; 27,5 ; 30,8 ; 39,5 ; 65,5 ; 119,0 ; 124,4 ; 124,8 ; 129,5 ; 132,7 ; 137,2 ; 141,4 ; 149,5 ; 152,8 ; 164,1 ; 176,5 ; 192,7.
Microanalyse : (C₂₀H₂₃NO₅)
Théorie: C 67,21%; H 6,49%; N 3,92%
Exp.: C 67,05%; H 6,52%; N 3,90%

### 4^{ème}étape : préparation de l'acide 8- (2,2-diméthylpropionyloxy)-7-butoxycarbonyl-quinoléine-2-carboxylique (5).

Un mélange de l'aldéhyde précédent 4 (étape 3) (3,5 g, 10 mmol) de dihydrogénophosphate de sodium (1,7 g, 12 mmol), de 2-méthyl-2-butène (6,1 ml, 57 mmol) et de chlorite de sodium (2,6 g, 29 mmol) est agité à 20 °C dans 160 ml de mélange *ter*-butanol / eau 5 / 1 et 30 ml de dichlorométhane pendant 12 heures. Le milieu réactionnel est concentré sous pression réduite. Le résidu est chromatographié sur colonne de silice en éluant avec de l'acétate d'éthyle pur pour donner 2,4 g (64%) d'acide 8-(2,2-diméthylpropionyloxy)-7-butoxycarbonyl-quinoléine-2-carboxylique 5 sous forme de cristaux blancs.
F = 158 °C
Rf = 0,41 (acétate d'éthyle)
IR (pur, cm⁻¹) v: 1693 ; 1730; 1753.
RMN¹H (δ en ppm, CDCl₃, 200MHz) 0,98 (t, *J*=7,2 Hz, 3H); 1,46(hex, *J*=7,2 Hz, 2H); 1,50(s, 9H); 1,78(quint, *J*=6,8 Hz, 2H) ; 4,35(t, *J*=6,8 Hz, 2H) ; 7,79 (d, *J*=8,7 Hz, 1H) ; 8,13 (d, *J*=8,7 Hz, 1H) ; 8,29(d, *J*=8,5 Hz, 1H) ; 8,40 (d, *J*=8,5 Hz, 1H) ; 9,27(s élargi, 1H)
Microanalyse : (C₂₀H₂₃NO₆)
Théorie : C 64,33%; H 6,21%; N 3,75%
Exp.: C 64,33%; H 6,35%; N 3,70%

### 5^{ème} étape : préparation du 8-(2,2-diméthylpropionyloxy)-7-butoxycarbonyl-quinoléine-carboxylate de 2,5-dioxopyrrolidin-1-yle (6)

Un mélange de l'acide précédent 5 (étape 4) (1,2 g, 3,2 mmol), de dicyclohexylcarbodiimide (0,7 g, 3,2 mmol) et de N-hydroxy-succinimide (0,4 g, 3,2 mmol) dans 12 ml de dichlorométhane est agité à 20 °C pendant 12 heures. Le milieu réactionnel est concentré sous pression réduite. Le résidu est chromatographié sur colonne de silice en éluant par un mélange cyclohexane / acétate d'éthyle 2 / 1 pour donner 1,2 g (80%) de cristaux blancs de 8-(2,2-diméthylpropionyloxy)-7-butoxycarbonyl-quinoléine-carboxylate de 2,5-dioxo-pyrrolidin-1-yle 6.
F = 143 °C
Rf = 0,71 (acétate d'éthyle)
IR (pur, cm⁻¹) v: 1709; 1735; 1736.
RMN¹H (δ en ppm, CDCl₃, 200MHz) 0,90 (t, *J*=7,4 Hz, 3H) ; 1,42 (hex, *J*=7, 4 Hz, 2H) ; 1,50 (s, 9H); 1,78 (quint, *J*=7,0 Hz, 2H) ; 2,91 (s, 4H); 4,38 (t, *J*=7,0 Hz, 2H) ; 7,76 (d, *J*=8, 8 Hz, 1H) ; 8,17(d, *J*=8,8 Hz, 1H) ; 8,22 (d, *J*=8,6 Hz, 1H) ; 8,34(d, *J*=8,6 Hz, 1H)
Microanalyse : (C₂₄H₂₆N₂O₈)
Théorise: C 61,27%; H 5,57%; N 5,95%
Exp.: C 61,39%; H 5,75%; N 6,02%

### 6^{ème} étape : préparation du 2-(3,4-dihydroxy-benzylcarbamoyl)-8-(2,2-diméthyl-propionyloxy)-quinoléine-7-carboxylate de n-butyle (7)

Un mélange du produit précédent 6 (étape 5) (1,4 g, 2,9 mmol) et du paratoluènesulfonate de 3,4-dihydroxybenzylamine (0,9 g, 2,9 mmol) dans .10 ml de pyridine est agité à 20 °C pendant 12 heures. Le mélange est concentré sous pression réduite, et 10 ml d'eau sont ajoutés. Le mélange est acidifié à pH 4 avec une solution d'acide chlorhydrique 2 N, puis 20 ml de dichlorométhane sont ajoutés. Les phases sont séparées et la phase aqueuse extraite au dichlorométhane. Les phases organiques rassemblées sont séchées sur sulfate de magnésium et concentrées sous pression réduite. Le résidu est chromatographié sur colonne de silice en éluant par un mélange cyclohexane / acétate d'éthyle 2 / 1 pour donner 1,1 g (80%) de 2-(3,4-dihydroxy-benzylcarbamoyl)-8-(2,2-diméthyl-propionyloxy)-quinoléine-7-carboxylate de n-butyle 7. Solide beige.
F = 189 °C
Rf = 0,73 (acétate d'éthyle)
IR (pur, cm⁻¹) ν: 1657; 1719; 1754; 3181; 3369; 3494.
RMN¹H (δ en ppm, DMSO-d6, 200MHz) 0,98 (t, *J*=7,4 Hz, 3H); 1,41 (s, 9H) ; 1,45 (hex, *J*=7,4 Hz, 2H); 1,75 (quint, *J*=7,0 Hz, 2H) ; 4,35 (t, *J*=7,0 Hz, 2H); 4,51 (d, *J*=5,7 Hz, 2H); 6,07 (t, *J*=5,7 Hz, 1H mobile); 6,63(dd, *J*=1,8 Hz, *J*=8, 0 Hz, 1H) ; 6,77 (d, *J*=8,0 Hz, 1H) ; 6,80 (d, *J*=1,8 Hz, 1H) ; 7,70 (d, J=8,5 Hz, 1H); 8,05(d, *J*=8,5 Hz, 1H), 8,25(d, *J*=8,5 Hz, 1H),
8,32(d, *J*=8,5 Hz, 1H)
Microanalyse : (C₂₇H₃₀N₂O₇)
Théorie : C 65,58%; H 6,11%; N 5,66%
Exp. : C 65,57%; H 6,13%; N 5,56%

### 7^{ème} étape : préparation de l'acide 2-(3,4-dihydroxybenzylcarbamoyl)-8-hydroxy-quinoléine-7-carboxylique (8)

Un mélange de l'amide précédent 7 (étape 6) (0,5 g, 1,0 mmol) et de 6,5 ml d'une solution d'hydroxyde de sodium 3 N dans 10 ml de méthanol est agité à 20 °C pendant 3 heures. Le pH est ajusté à 4 avec une solution d'acide sulfurique 1 N. Le mélange réactionnel est concentré sous pression réduite, puis séché sous vide sur anhydride phosphorique. Le résidu est repris dans 10 ml d'éthanol anhydre à chaud et le sulfate de sodium insoluble est filtré. Le filtrat est concentré sous pression réduite, puis repris dans 2 ml d'éthanol anhydre. Le milieu est refroidi : un solide jaunâtre cristallise. Ce dernier est lavé au dichlorométhane, puis séché sous vide sur anhydride phosphorique pour donner 250 mg d'acide 8 sous la forme d'un solide brun.
F = 232 °C (décomposition)
IR (pur, cm⁻¹) v: 1645; 1670; 3249
RMN¹H (δ en ppm, DMSO-d6, 200MHz) 4,40 (d, *J*=6,2 Hz, 2H) ; 6,60(dd, *J*=1,75 Hz, *J*=8,04 Hz, 1H); 6,65(d, *J*=8,0 Hz, 1H); 6,75(d, *J*=1,7 Hz, 1H); 7,22(d, *J*=8,5 Hz, 1H); 7,88(d, *J*=8,5 Hz, 1H), 8,15(d, *J*=8, 5 Hz, 1H) , 8,40 (d, *J*=8,6 Hz, 1H) , 8,72 (s élargi, 1H), 8,85(s élargi, 1H), 9,27(t, J=6,2 Hz, 1H),
Microanalyse : (C₁₈H₁₄N₂O₆, 1,5 H₂O)
Théorie: C 56,69%; H 4,49%; N 7,35%
Exp.: C 57,13%; H 4,17%; N 7,33%

### 8^{ème} étape : préparation du 2 - (3,4-dihydroxy-benzylcarbamoyl)-8-hydroxy-quinoléine-7-carboxylate de sodium

Un mélange de l'amide précédent 8 (étape 7) (20 mg, 0,06 mmol) et 0,66 ml d'une solution d'hydroxyde de sodium 0,1 N sont agités à 20 °C pendant 10 minutes. Le sel de sodium obtenu est lyophilisé pour donner quantitativement 22 mg de solide marron foncé.

### Autres produits :

### Préparation de l'acide 2-(2,4-dihydroxy-benzylcarbamoyl)-8-hydroxy-quinoléine-7-carboxylique

On met en oeuvre la même méthode de préparation que celle décrite pour l'acide (8) .

F = 196 °C (décomposition)
IR (pur, cm⁻¹) ν: 1624; 1638; 3311.
RMN¹H (δ en ppm, DMSO-*d6*, 200MHz) 4,45 (d, *J*=5,8 Hz, 2H) ; 6,20(dd, *J*=2,4 Hz, *J*=8,2 Hz, 1H); 6,38(d, *J*=2,4 Hz, 1H); 7,05 (d, *J*=8,2 Hz, 1H) ; 7,34(d, *J*=8,7 Hz, 1H); 7,95(d, *J*=8,6 Hz, 1H); 8,22(d, *J*=8,5 Hz, 1H); 8,50(d, *J*=8,6 Hz, 1H); 9,17(s élargi, 1H); 9,28(t, *J*=5,8 Hz, 1H); 9,58(s élargi, 1H)
Microanalyse : (C₁₈H₁₄N₂O₆, 2 H₂O)
Théorie: C 55,39%; H 4,65%; N 7,18%
Exp.: C 55,97%; H 4,57%; N 6,73%

### Préparation de l'acide 2-(2,3,4-trihydroxy-benzylcarbamoyl)-8-hydroxy-quinoléine-7-carboxylique

Même méthode de préparation que pour l'acide (8).
F = 208 °C (décomposition)
IR (pur, cm⁻¹) v:1625; 1659; 3300.
RMN¹H (δ en ppm, DMSO-d6, 200MHz) 4,45 (d, *J*=6,0 Hz, 2H); 6,29(d, *J*=8,5 Hz, 1H) ; 6,53(d, *J*=8,5 Hz, 1H) ; 7,40(d, *J*=8,8 Hz, 1H); 7,92(d, *J*=8,6 Hz, 1H); 8,28(d, *J*=8,6 Hz, 1H); 8,52(d, *J*=8,5 Hz, 1H); 8,72(s élargi, 1H); 8,89(s élargi, 1H); 9,50(t, *J*=6,0 Hz, 1H)
Microanalyse : (C₁₈H₁₄N₂O₇, H₂O)
Théorie : C 55,67%; H 4,15%; N 7,21%
Exp.: C 56,54%; H 4,66%; N 6,31%

### III. Etude des propriétés antivirales dirigées contre les intégrases rétrovirales des dérivés selon l'invention

On rapporte ci-après, à titre illustratif, les résultats obtenus avec la 2-(3-,4-dihydroxy-benzylcarbamoyl)-8-hydroxyquinoléine-7-carboxylate de sodium, la 2-(2,3,4-trihydroxybenzylcarbamoyl)-8-hydroxy-quinoléine-7-carboxylate de sodium et la 2-(2,4-dihydroxy-benzylcarbamoyl)-8-hydroxy-quinoléine-7-carboxylate de sodium. Ces composés seront désignés respectivement par BioA53, BioA162, BioA163.

### Exemple 1: Inhibition de l'activité in vitro

L'activité de l'intégrase du VIH-1 est mesurée par les tests suivants :
1. L'activité endonucléolytique de la protéine recombinante est testée sur un oligonucléotide double brin de 21 paires de bases ; radiomarqué à l'extrémité 5'. L'activité de l'intégrase se traduit par l'élimination du dinucléotide à l'extrémité 3'.
2. Le test de transfert de brins est réalisé avec un oligonucléotide double-brin de 21 paires de bases mimant l'extrémité de l'ADN viral dont le dinucléotide 3' terminal a été supprimé. L'activité de la protéine se traduit par l'insertion covalente de cet oligonucléotide dans un oligonucléotide homologue.
3. Le test de désintégration est réalisé avec un substrat mimant la structure de l'ADN viral intégré. On mesure la quantité d'ADN excisé par l'intégrase. Ce dernier test ne mesure que l'activité catalytique de la protéine, excluant son activité de fixation à l'ADN.

Les activités enzymatiques sont testées en présence de manganèse et/ou de magnésium.

Les composés selon la présente invention inhibent les activités enzymatiques, aussi bien en présence de manganèse que de magnésium.

### Exemple 2 : Inhibition de la réplication du virus de l'immunodéficience Humaine (VIH-1)

Deux tests sont utilisés pour déterminer l'efficacité antivirale du composé selon l'invention.

Le premier test consiste à mettre en contact des cellules d'une lignée lymphocytaire établie, cellules CEM, avec un surnageant de cellules infectées contenant les virions infectieux. Le test est réalisé de la manière suivante : les cellules CEM, cultivées en suspension dans du milieu RPMI complémenté avec 10% de sérum de veau foetal, sont infectées par un surnageant viral avec une multiplicité d'infection de 0,5. Après deux heures d'infection, les cellules sont lavées deux fois avec du RPMI de manière à éliminer les particules virales résiduelles. Les cellules sont alors replacées dans du milieu RPMI contenant le composé selon l'invention. La charge virale est évaluée après 72 heures de culture. Celle-ci est quantifiée par les deux méthodes suivantes :
1. La quantité de protéine virale p24 est déterminée par un test ELISA.
2. La charge en virus infectieux est estimée par infection des cellules HeLa β-gal CD4+. L'efficacité d'intégration rétrovirale est estimée par un test colorimétrique (CPRG).

Le second test consiste à mettre en contact des cellules HeLa β-gal CD4+ (cellules fibroblastiques humaines contenant le gène de la β-galactosidase sous contrôle transcriptionnel du transactivateur viral Tar et exprimant le récepteur membranaire CD4) avec le surnageant de cellules transfectées par le plasmide pNL43, contenant des virions infectieux, et les composés selon l'invention. En pratique, les cellules HeLa β-gal CD4+ sont cultivées en plaques 96 puits dans du milieu DMEM complémenté avec 10% de sérum de veau foetal, mises en contact avec 5ng de p24 (issus d'une transfection de cellules 293T avec le plasmide pNL43) et des dilutions du composé selon l'invention. Après 48 heures, les cellules sont lysées et la β-galactosidase est dosée par coloration au CPRG.

Les résultats obtenus sont représentés sur les figures 1A (-◆- BioA53) et 1B (-◆- BioA53 ; -■- BioA162 -▲- BioAl63).

Dans les deux tests d'activité, les composés selon l'invention montrent un effet protecteur contre l'infection par le virus VIH-1. En effet, cet effet protecteur se traduit par une inhibition de la production de particules virales dans le premier test et une inhibition de la coloration CPRG dans le second test, à des concentrations micromolaires, voire submicromolaires. Concernant les efficacités antirétrovirales, les IC50 varient de 0,3 µM à 4µM.

### Exemple 3 Cytotoxicité des composés

La toxicité des composés est évaluée par un test de biotransformation de MTT⁻ en formazan par les déshydrogénases mitochodriales cellulaires.

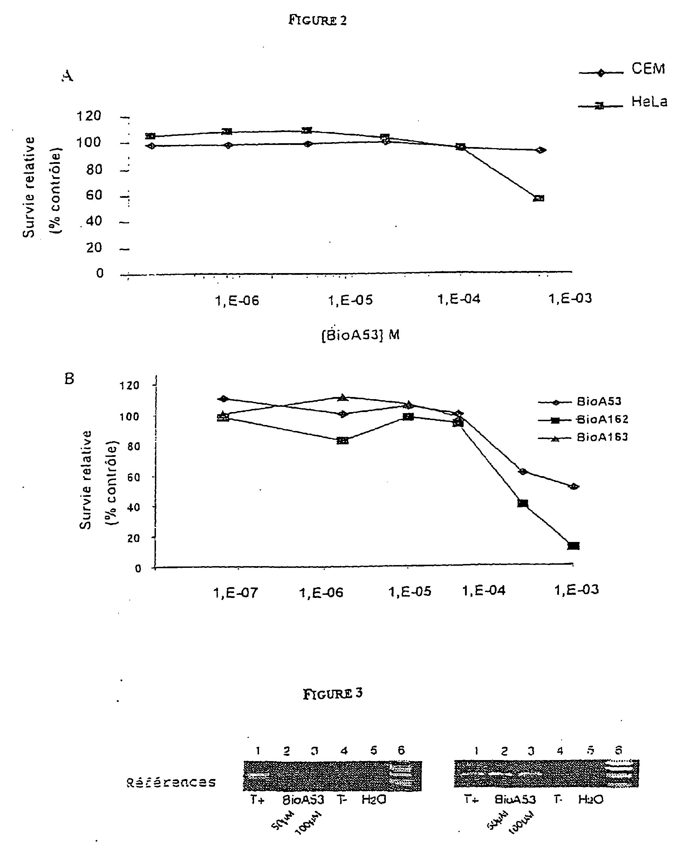

On constate que le composé testé est de plus dépourvu de cytotoxicité à 500µM à la fois sur des cellules adhérentes (HeLa) et en suspension (CEM) comme illustré par les résultats rapportés sur les figures 2A (-◆- CEM ; -■- HeLa) et 2B (-◆- BioA53 ; -■- BioA162 ; -▲- BioA163) .

### Exemple 4 : Inhibition de l'intégration de l'ADN viral dans le génome hôte

Ce test consiste à mettre en contact des cellules CEM avec le surnageant de cellules infectées contenant les virions infectieux. Après 2 heures, les cellules sont lavées et replacées dans du milieu RPMI contenant le composé selon l'invention durant 24 heures (BioA53).

L'ADN des cellules est alors extrait en utilisant un kit de préparation d'ADN génomique (QiaBlood, Qiagen^{™}). Cet ADN est la cible d'amplifications par PCR visant à évaluer, d'une part, la quantité d'ADN viral total présent dans la solution, et, d'autre part, la quantité d'ADN viral intégré dans le génome cellulaire. Cette dernière amplification a lieu en deux temps : la première étape utilise une amorce de PCR homologue à une séquence de l'ADN rétroviral ; la seconde amorce est homologue aux séquences génomiques répétées Alu. La seconde étape est une PCR nichée permettant de révéler l'ADN rétroviral.

La quantité d'ADN est évaluée par densitométrie après séparation des produits d'amplification en gel d'agarose.

Le composé selon la présente invention montre une diminution d'au moins 80% de la quantité d'ADN intégré pour une concentration de 50µM et une inhibition de la totalité de l'ADN intégré à 100µM (figure 3A) alors que la quantité d'ADN viral total n'est pas affectée par le traitement de cellules avec le composé (Figure 3B).

### Exemple 5 : préparation de comprimés

On prépare des comprimés renfermant BioA53 en procédant comme suit :

On ajoute à une solution aqueuse d'hydroxypropyl cellulose une quantité thérapeutiquement active du composé BioA53, d'amidon de mais et de cellulose microcristalline. On fait passer le mélange à travers un tamis pour produire des granules. Après séchage, on mélange avec du stéarate de magnésium et on formule en comprimés de 250 mg selon les techniques habituelles.

### RÉFÉRENCES BIBLIOGRAPHIQUES

1- Meek, W. H.; Fuschman, C. H. J.; J. Chem. Eng. Data 1969, 14, 388-391.
2- Bader, A.R. ; Kontowicz, A.D. J. Am. Chem. Soc. 1953, 75, 5416-5417.
3- Epstein, J., Michel, H.O., Rosenblatt.D.H., Plapinger, R.E., Stephani, R.A., Cook, E. J. Am. Chem. Soc. 1964, 86, 4959-4963.

## Revendications

1. Dérivés de l'acide 2-carbamoyl-8-hydroxyquinoléine 7-carboxylique et leurs sels pharmaceutiquement acceptables, **caractérisés en ce qu'**ils répondent à la formule générale 1 : 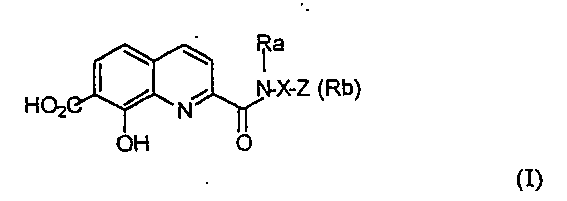 dans laquelle
- X représente une chaîne alkyle-(CH₂)ₙ- dans laquelle n est égal à 0, 1 ou 2, ou O, ou N,
- Z représente un cycle aromatique pouvant comporter des hétéroatomes choisis parmi O, N ou S, en substitution des atomes de carbone constituant ledit cycle aromatique, ce cycle pouvant ou non être substitué par Rb,
- Rb représente 1 à 3 substituants identiques ou différents, choisis parmi les groupements -OH, -OR,-COOH, -COOR, -COH, -COR, -NH₂, -NH(R), -NH(R,R') , -SH, -.SR et CN,
- Ra représente un atome d'hydrogène ou un groupe (CH₂)ₙ, -Y, pour lequel n'est égal à 0, 1, 2 ou 3 et Y représente -CH₃, -COOH, -COOR, -CN, -OH, -OR, SR, ou un groupement aryle éventuellement substitué par Rb,
- R et R', identiques ou différents, représentent une chaîne alkyle linéaire ou ramifiée de 1 à 4 atomes de carbone, et
- leurs sels pharmaceutiquement acceptables.

2. Dérivés de l'acide 2-carbamoyl-8-hydroxyquinoléine-7-carboxylique choisis parmi l'acide 2-(3,4-dihydroxybenzylcarbamoyl)-8-hydroxy-quinoléine-7-carboxylique, l'acide 2-(2,4-dihydroxy-benzylcarbamoyl)-8-hydroxy-quinoléine-7-carboxylique, l'acide 2-(2,3,4-trihydroxy-benzylcarbamoyl)-8-hydroxyquinoléine-7-carboxylique et leurs sels de sodium.

3. Procédé de synthèse de dérivés selon la revendication 1, **caractérisé en ce qu'**il comprend la réaction de la quinoléinecarboxylate du succinimidyle de formule II 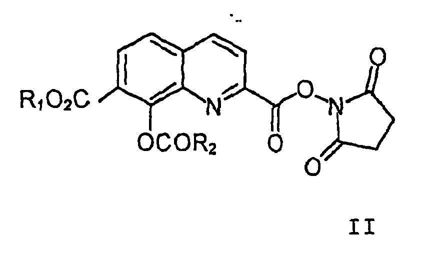 dans laquelle R₁ et R₂, identiques ou différents, représentent des radicaux alkyle de 1 à 4C, avec une amine de formule III 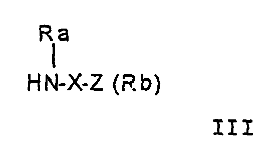 le cas échéant sous forme de sel, suivie d'une saponification, et le cas échéant d'une acidification.

4. Procédé selon la revendication 3, **caractérisé en ce que**, pour préparer des dérivés de formule IA avec un bras espaceur amide, 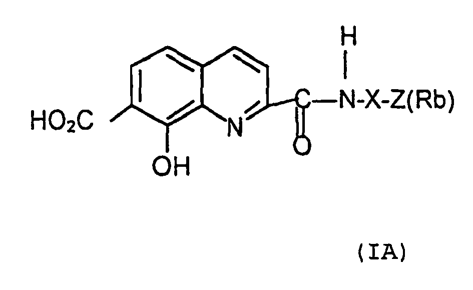 dans laquelle Rb et Z sont tels que définis ci-dessus, et X représente un groupe -(CH₂)ₙ- comme défini plus haut, on fait réagir la quinoléinecarboxylate de succinimidyle de formule II avec un sel d'amine de formule IV suivante :
[H₃N-X-Z(Rb)] ⁺A⁻ (IV)
dans laquelle Z et Rb sont tels que définis ci-dessus, X = (CH₂) ₙ comme défini plus haut, et A- est un anion de sel organique, notamment un p-toluènesulfonate, les dérivés d'amide étant récupérés par saponification, suivie d'une acidification.

5. Procédé selon la revendication 3, **caractérisé en ce que** pour préparer des dérivés de formule IB avec un bras espaceur substitué , 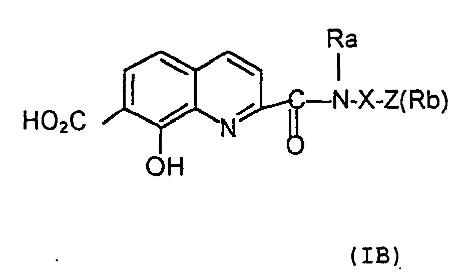 dans laquelle Rb et Z sont tels que définis ci-dessus, et X représente un groupe - (CH₂) ₙ- connu défini plus haut, on fait réagir le dérivé de formule II avec une amine de formule V suivante : 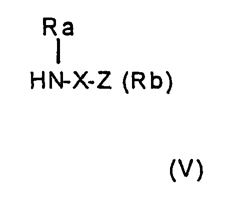 dans laquelle les substituants sont tels que définis ci-dessus, cette réaction étant suivie d'une saponification.

6. Procédé selon la revendication 3, **caractérisé en ce que** pour préparer des dérivés de formule IC avec un bras espaceur hydrazide, 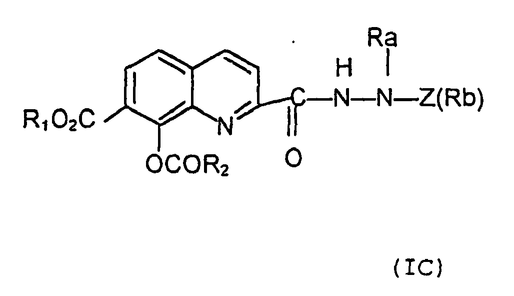 dans laquelle les différents substituants sont tels que définis ci-dessus, on fait réagir le composé de formule II avec l'hydrazine de formule VI suivante :

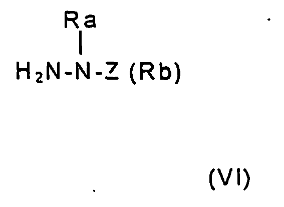

7. Procédé selon la revendication 3, **caractérisé en ce que** pour préparer des dérivés de formule ID avec un bras espaceur alcoxyamide 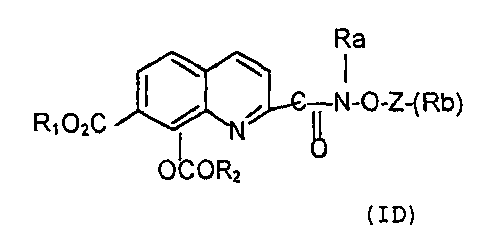 dans laquelle les différents substituants sont tels que définis ci-dessus, on fait réagir le composé de formule II avec l'hydroxylamine de formule VII suivante : 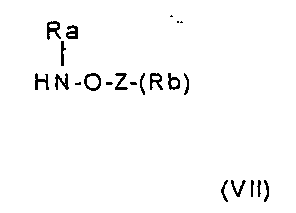 dans laquelle Ra, Rb et Z sont tels que définis ci-dessus.

8. Procédé selon la revendication 3, **caractérisé en ce que** les dérivés protégés de quinoléine de formule II sont obtenus à partir d'un hydroxy-acide de quinoléine de formule VIII :

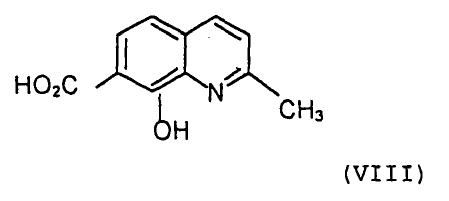

9. Procédé selon la revendication 8, **caractérisé par** la protection des groupes -COOH et -OH à l'aide de dérivés capables de générer des groupes protecteurs spécifiques par rapport aux fonctions concernées et pouvant être ensuite coupés sélectivement sans altérer le reste de la molécule.

10. Compositions pharmaceutiques, **caractérisées en ce qu'**elles comprennent une quantité efficace d'au moins un dérivé tel que défini dans l'une quelconque des revendications 1 ou 2 en association avec des véhicules pharmaceutiquement acceptables.

11. Dérivés selon l'une quelconque des revendications 1 ou 2 pour utilisation comme réactifs biologiques.

12. Dérivés selon l'une quelconque des revendications 1 ou 2 pour utilisation comme médicament antiviral.

13. Dérivés selon la revendication 12, tels que ledit médicament est inhibiteur des intégrases VIH-1, VIH-2, VIS et VSR.

14. Dérivés selon l'une quelconque des revendications 12 ou 13 tels que ledit médicament est destiné à traiter le VIH.

15. Combinaisons d'un dérivé selon la revendication 1 ou 2 avec d'autres médicaments anti-VIH.

16. Utilisation d'un dérivé selon la revendication 1 ou 2 pour la préparation d'un médicament pour traiter le VIH.

## Claims

1. 2-Carbamoyl-8-hydroxyquinoline-7-carboxylic acid derivatives and pharmaceutically acceptable salts thereof, **characterised in that** they correspond to the general formula I: 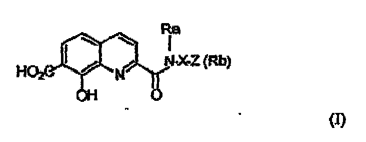 in which
- X represents an alkyl-(CH₂)ₙ - chain, in which n is 0, 1 or 2, O or N,
- Z represents an aromatic ring which can comprise heteroatoms selected from O, N and S substituting the carbon atoms constituting said aromatic ring, which ring may or may not be substituted by Rb,
- Rb represents from 1 to 3 identical or different substituents selected from the groups -OH, -OR, -COOH, -COOR, -COH, -COR, -NH₂, -NH(R), -NH(R,R'), -SH, -SR and CN,
- Ra represents a hydrogen atom or a group -(CH₂)_{n'}-Y wherein n' is 0, 1, 2 or 3 and Y represents -CH₃, -COOH, -COOR, -CN, - OH, -OR, SR, or an aryl group optionally substituted by Rb,
- R and R', which may be identical or different, represent a linear or branched alkyl chain having from 1 to 4 carbon atoms, and
- their pharmaceutically acceptable salts.

2. 2-Carbamoyl-8-hydroxyquinoline-7-carboxylic acid derivatives selected from 2-(3,4-dihydroxybenzylcarbamoyl)-8-hydroxyquinoline-7-carboxylic acid, 2-(2,4-dihydroxybenzylcarbamoyl)-8-hydroxyquinoline-7-carboxylic acid, 2-(2,3,4-trihydroxybenzylcarbamoyl)-8-hydroxyquinoline-7-carboxylic acid and their sodium salts.

3. Process for the synthesis of derivatives according to claim 1, **characterised in that** it comprises the reaction of the succinimidyl quinolinecarboxylate of formula II 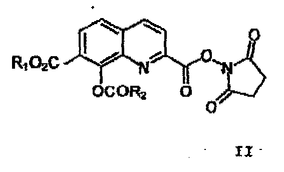 in which R₁ and R₂, which may be identical or different, represent C₁₋₄-alkyl radicals, with an amine of formula III 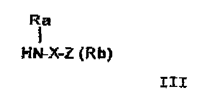 where appropriate in salt form, followed by saponification and, where appropriate, acidification.

4. Process according to claim 3, **characterised in that**, in order to prepare derivatives of formula IA having an amide spacer arm 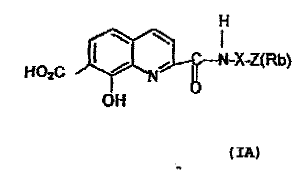 in which Rb and Z are as defined above and X represents a group -(CH₂)ₙ- as defined hereinbefore, the succinimidyl quinolinecarboxylate of formula II is reacted with an amine salt of formula IV below:
[H₃N-X-Z(Rb)]⁺A⁻ (IV)
in which Z and Rb are as defined above, X = (CH₂)ₙ as defined hereinbefore, and A⁻ is an anion of an organic salt, especially a p-toluenesulfonate, the amide derivatives being recovered by saponification, followed by acidification.

5. Process according to claim 3, **characterised in that**, in order to prepare derivatives of formula IB having a substituted spacer arm 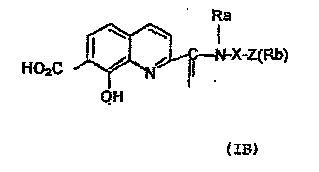 in which Rb and Z are as defined above and X represents a group -(CH₂)ₙ- as defined hereinbefore, the derivative of formula II is reacted with an amine of formula V below: 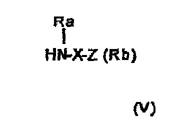 in which the substituents are as defined above, this reaction being followed by a saponification.

6. Process according to claim 3, **characterised in that**, in order to prepare derivatives of formula IC having a hydrazide spacer arm 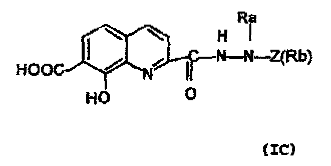 in which the various substituents are as defined above, the compound of formula II is reacted with the hydrazine of formula VI below:

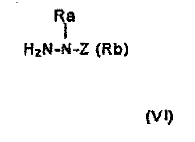

7. Process according to claim 3, **characterised in that**, in order to prepare derivatives of formula ID having an alkoxyamide spacer arm 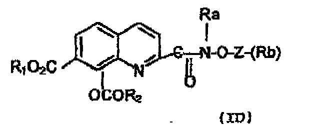 in which the various substituents are as defined above, the compound of formula II is reacted with the hydroxylamine of formula VII below: 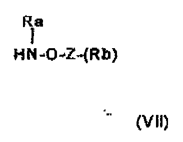 in which Ra, Rb and Z are as defined above.

8. Process according to claim 3, **characterised in that** the protected quinoline derivatives of formula II are obtained from a quinoline hydroxy acid of formula VIII:

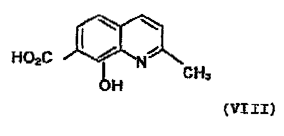

9. Process according to claim 8, **characterised in that** the - COOH and -OH groups are protected by means of derivatives capable of producing protecting groups which are specific in relation to the functions in question and which can subsequently be cleaved selectively without altering the remainder of the molecule.

10. Pharmaceutical compositions, **characterised in that** they comprise an effective amount of at least one derivative as defined in either claim 1 or claim 2, in association with pharmaceutically acceptable carriers.

11. Derivatives according to either claim 1 or claim 2 for use as biological reagents.

12. Derivatives according to either claim 1 or claim 2 for use as an antiviral medicament.

13. Derivatives according to claim 12, such that said medicament is an inhibitor of HIV-1, HIV-2, SIV and RSV integrases.

14. Derivatives according to either claim 12 or claim 13, such that said medicament is for the treatment of HIV.

15. Combinations of a derivative according to claim 1 or 2 with other anti-HIV medicaments.

16. Use of a derivative according to claim 1 or 2 in the preparation of a medicament for the treatment of HIV.

## Patentansprüche

1. Derivate der 2-Carbamoyl-8-hydroxychinolin-7-carbonsäure und deren pharmazeutisch geeignete Salze, **dadurch gekennzeichnet, dass** sie der allgemeinen Formel (I) entsprechen: 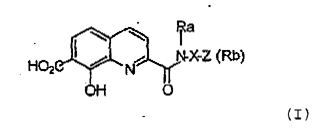 wobei
- X für eine Alkylkette -(CH₂)ₙ- steht, wobei n gleich 0, 1 oder 2 oder O oder N ist,
- Z für einen aromatischen Ring steht, der Heteroatome, die aus O, N oder S ausgewählt sind, als Substitution für die Kohlenstoffatome, die den aromatischen Ring bilden, aufweisen kann, wobei der Ring mit Rb substituiert werden kann oder nicht,
- Rb für 1 bis 3 identische oder unterschiedliche Substituenten steht, die aus den Gruppen -OH, -OR, -COOH, -COOR, -COH, -COR, -NH₂, -NH(R), -NH(R, R'), -SH, -SR und CN ausgewählt sind,
- Ra für ein Wasserstoffatom oder eine Gruppe -(CH₂)ₙ -Y steht, wobei n' gleich 0, 1, 2 oder 3 ist, und Y für -CH₃, -COOH, -COOR, -CN, -OH, -OR, SR oder eine Aryl-Gruppe steht, die gegebenenfalls mit Rb substituiert wird,
- R und R', die identisch oder unterschiedlich sind, für eine lineare oder verzweigte Alkylkette mit 1 bis 4 Kohlenstoffatomen stehen, und
- ihre pharmazeutisch geeigneten Salze.

2. Derivate der 2-Carbamoyl-8-hydroxychinolin-7-carbonsäure, die aus der 2-(3,4-Dihydroxybenzylcarbamoyl)-8-hydroxychinolin-7-carbonsäure, der 2-(2,4-Dihydroxybenzylcarbamoyl)-8-hydroxychinolin-7-carbonsäure, der 2-(2,3,4-trihydroxy-benzylcarbamoyl)-8-hydroxychinolin-7-carbonsäure und ihren Natriumsalzen ausgewählt sind.

3. Verfahren für die Synthese von Derivaten gemäß Anspruch 1, **dadurch gekennzeichnet, dass** es aufweist: die Umsetzung des Succinimidylchinolincarboxylats des der Formel II, 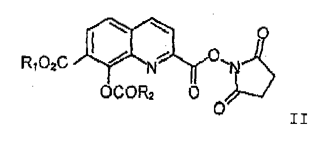 wobei R₁ und R₂, die identisch oder unterschiedlich sind, für Alkylreste mit 1 bis 4 C stehen, mit einem Amin der Formel III, 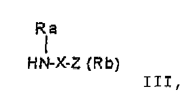 gegebenenfalls in Form eines Salzes, gefolgt von einer Verseifung und gegebenenfalls einer Ansäuerung.

4. Verfahren gemäß Anspruch 3, **dadurch gekennzeichnet, dass** zur Herstellung der Derivate der Formel IA mit einem Amid-Spacer-Arm, 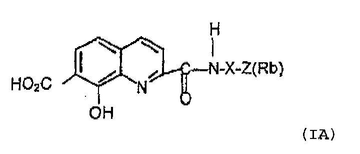 wobei Rb und Z wie oben definiert sind und X für eine wie oben definierte Gruppe -(CH₂)ₙ- steht, das Succinimidylchinolincarboxylat der Formel II mit einem Aminsalz der folgenden Formel IV umgesetzt wird:
[H₃N-X-Z (Rb)]⁺A⁻ (IV)
wobei Z und Rb wie oben definiert sind, X = (CH₂)ₙ, wie oben definiert ist, und A⁻ ein Anion eines organischen Salzes, insbesondere ein p-Toluolsulfonat ist, wobei die Amid-Derivate durch Verseifung gefolgt von einer Ansäuerung wiedergewonnen werden.

5. Verfahren gemäß Anspruch 3, **dadurch gekennzeichnet, dass** zur Herstellung der Derivate der Formel IB mit einem substituierten Spacer-Arm, 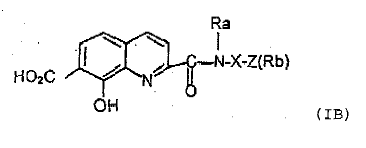 wobei Rb und Z wie oben definiert sind und X für eine Gruppe -(CH₂)ₙ- steht, die bekannt und oben definiert ist, das Derivat der Formel II mit einem Amin der folgenden Formel V umgesetzt wird: 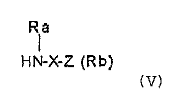 wobei die Substituenten wie oben definiert sind, wobei auf die Umsetzung eine Verseifung folgt.

6. Verfahren gemäß Anspruch 3, **dadurch gekennzeichnet, dass** zur Herstellung der Derivate der Formel IC mit einem Hydrazid-Spacer-Arm, 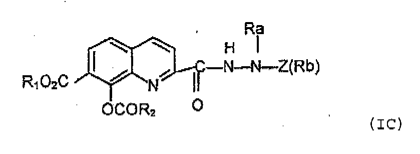 wobei die verschiedenen Substituenten wie oben definiert sind, die Verbindung der Formel VII mit dem Hydrazin der folgenden Formel VI umgesetzt wird:

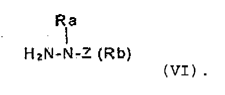

7. Verfahren gemäß Anspruch 3, **dadurch gekennzeichnet, dass** zur Herstellung der Derivate der Formel ID mit einem Alkoxyamid-Spacer-Arm 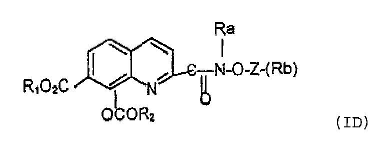 wobei die verschiedenen Substituenten wie oben definiert sind, die Verbindung der Formel II mit dem Hydroxylamin der folgenden Formel VII umgesetzt wird, 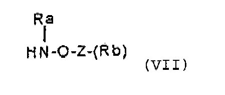 wobei Ra, Rb und Z wie weiter oben definiert sind.

8. Verfahren gemäß Anspruch 3, **dadurch gekennzeichnet, dass** die geschützten Chinolin-Derivate der Formel II ausgehend von einer Hydroxychinolinsäure der Formel VIII erhalten werden:

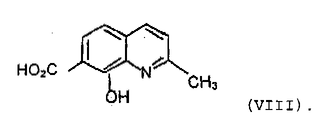

9. Verfahren gemäß Anspruch 8, **gekennzeichnet durch** das Schützen der Gruppen -COOH und -OH mit Hilfe von Derivaten, die geeignet sind, die Schutzgruppen, die relativ zu den jeweiligen Funktionen spezifisch sind und die anschließend selektiv abgespaltet werden können, ohne den Rest des Moleküls zu beeinträchtigen, zu bilden.

10. Pharmazeutische Zusammensetzungen, **dadurch gekennzeichnet, dass** sie eine wirksame Menge von mindestens einem wie in einem der Ansprüche 1 oder 2 definierten Derivat in Verbindung mit pharmazeutisch geeigneten Trägern aufweisen.

11. Derivate gemäß einem der Ansprüche 1 oder 2 für die Verwendung als biologische Reagenzien.

12. Derivate gemäß einem der Ansprüche 1 oder 2 für die Verwendung als antivirales Medikament.

13. Derivate gemäß Anspruch 12, wobei das Medikament ein HIV-1-, HIV-2-, SIV- und RSV-Integrasehemmer ist.

14. Derivate gemäß einem der Ansprüche 12 oder 13, wobei das Medikament zur Behandlung von HIV bestimmt ist.

15. Kombinationen eines Derivates gemäß Anspruch 1 oder 2 mit anderen Anti-HIV-Medikamenten.

16. Verwendung eines Derivates gemäß Anspruch 1 oder 2 für die Herstellung eines Medikaments zur Behandlung von HIV.
